Europäisches Patentamt

European Patent Office

Office européen des brevets

(19)

(11) Veröffentlichungsnummer: **0 120 262**
**B1**

(12) **EUROPÄISCHE PATENTSCHRIFT**

(45) Veröffentlichungstag der Patentschrift:
**13.07.88**

(21) Anmeldenummer: **84101528.2**

(22) Anmeldetag: **15.02.84**

(51) Int. Cl.⁴: **A 61 K 9/08, A 61 K 31/70,
A 61 K 31/34, A 61 K 47/00**

(54) **Pharmazeutische Zubereitung mit Retardwirkung.**

(30) Priorität: **18.02.83 DE 3305689**

(43) Veröffentlichungstag der Anmeldung:
**03.10.84 Patentblatt 84/40**

(45) Bekanntmachung des Hinweises auf die Patenterteilung:
**13.07.88 Patentblatt 88/28**

(84) Benannte Vertragsstaaten:
**AT BE CH DE FR GB IT LI LU NL SE**

(56) Entgegenhaltungen:
**DE - A - 2 621 214
GB - A - 2 081 582**

**CHEMICAL ABSTRACTS, Band 94, Nr. 4, Januar 1981,
Seite 312, nr. 20418m, Columbus, Ohio, US; & JP - A - 80
98 112 (SUMITOMO CHEMICAL CO., LTD.) 25.07.1980**

(73) Patentinhaber: **Heinrich Mack Nachf., Postfach 140,
D-7918 Illertissen (DE)**

(72) Erfinder: **Fries, Walter, Einsteinring 25, D-7918 Illertissen
(DE)**

(74) Vertreter: **Lederer, Franz, Dr. et al, Van der Werth,
Lederer & Riederer Patentanwälte
Lucile-Grahn-Strasse 22, D-8000 München 80 (DE)**

**Beschreibung**

Die Erfindung betrifft eine Isosorbiddinitrat als Wirkstoff enthaltende pharmazeutische Zubereitung mit Retardwirkung für die topische Anwendung.

Isosorbiddinitrat ist ein bekannter Wirkstoff zur Behandlung koronarer Durchblutungsstörungen. Seit langem ist man bemüht Anwendungsformen für diesen Wirkstoff zu finden, wobei der Wirkstoff allmählich von dem Organismus resorbiert wird, so dass die Präparate für eine Langzeittherapie geeignet sind.

Bei einem bekannten Handelspräparat wird die kontinuierliche Freigabe der Wirksubstanz durch eine besondere Pelletierung erreicht. Bei der oralen Verabreichung solcher Präparate in Kapselform werden gleichmässige, über mindestens 8 Stunden anhaltende hohe Blutspiegelwerte an dem Wirkstoff erzielt, der im Plasma noch 12 Stunden nach oraler Verabreichung nachweisbar ist.

Aus der US-PS 4 112 115 ist ein topisch anzuwendendes Präparat zur Minderung von Spasmen in Beuge- und Streckmuskeln bekannt, bei welchem der Nitrat-Wirkstoff als Lösung in einem pharmazeutisch annehmbaren. Träger vorliegt. Als Träger werden wässriges Äthanol und wässriges Isopropanol empfohlen, jedoch sollen auch Hautcreme und hydrophile Salben als Träger verwendet werden können.

Aus der DE-OS 2 924 005 ist eine pharmazeutische Zubereitung mit einem Gehalt an Isosorbiddinitrat bekannt, die neben dem Wirkstoff ein Lösungsmittel für Isosorbiddinitrat, einen Salbenkonsistenzgeber, Wasser, gegebenenfalls einen Emulgator und gegebenenfalls weitere übliche Zusatzstoffe wie Konservierungsmittel, Antioxidantien, pH-Regulatoren und/oder Duftstoffe enthält. Diese bekannten pharmazeutischen Zubereitungen enthalten eine beträchtliche Menge an Wasser; es soll das Gewichtsverhältnis von Wasser : Konsistenzgeber : Lösungsmittel 30–80 : 3–35 : 3–35 betragen. Bei dieser bekannten pharmazeutischen Zubereitung liegt das Isosorbiddinitrat teilweise in gelöstem, teilweise in ungelöstem Zustand vor; durch einen ausreichend grossen Anteil an ungelöstem Isosorbiddinitrat soll eine lange Wirkungsdauer erreicht werden. Bei dieser Zubereitungsform besteht jedoch die Gefahr, dass die auf der Haut verbleibenden noch nicht gelösten Wirkstoffkristalle durch die Wäsche abgewischt werden und dann für die allmähliche Lösung und Resorption nicht mehr zur Verfügung stehen. Demgegenüber schafft die vorliegende Erfindung eine flüssige Zubereitung, in der der Wirkstoff vollständig gelöst ist und die sofort in die Haut eindringt, dadurch nach der Applikation nicht mehr versehentlich entfernt werden kann, dennoch jedoch eine hervorragende Retardwirkung bringt.

Die erfindungsgemässe pharmazeutische Zubereitung mit einem Gehalt an Isosorbiddinitrat als Wirkstoff ist dadurch gekennzeichnet, dass sie

a) ein flüssiges lipophiles Lösungsmittel für Isosorbiddinitrat und

b) ein flüssiges hydrophiles Lösungsmittel für Isosorbiddinitrat in einem Gewichtsverhältnis von a : b von 0,8–0,2, vorzugsweise von 0,5–0,3, enthält.

Die zur Anwendung in der vorliegenden Erfindung geeigneten flüssigen lipophilen und hydrophilen Lösungsmittel sind solche, in denen Isosorbiddinitrat zu wenigstens 5 Gew.-%, vorzugsweise zu wenigstens 8 Gew.-%, insbesondere zu wenigstens 10 Gew.-% löslich ist. Die erfindungsgemässe pharmazeutische Zubereitung soll das Isosorbiddinitrat in einer Menge von 5–25 Gew.-%, vorzugsweise von 8–15 Gew.-% enthalten.

Geeignete flüssige lipophile Lösungsmittel sind Alkohole, z.B. solche enthaltend 8–12 Kohlenstoffatome, wie z.B. n-Decylalkohol; Äther, z.B. solche enthaltend 6–10 Kohlenstoffatome, wie Isosorbiddimethyläther; Ester von niederen Fettsäuren, z.B. solche enthaltend 4–10 Kohlenstoffatome, wie Glycerintriacetat; flüssige Triglyceride, z.B. solche von Fettsäuren enthaltend 6–18 Kohlenstoffatome, wie gesättigte Pflanzenfettsäuren mittlerer Kettenlänge; flüssige lipophile Ester von höheren Fettsäuren, z.B. solche enthaltend 10–18 Kohlenstoffatome, und einwertigen aliphatischen Alkoholen, z.B. solchen enthaltend 2–4 Kohlenstoffatome, wie Isopropylmyristat, Isobutylstearat, Isobutylpalmitat, Isopropylstearat oder Isopropylpalmitat; flüssige Ester von Dicarbonsäuren, z.B. solchen enthaltend 6–10 Kohlenstoffatome und von einwertigen Alkoholen, z.B. solchen enthaltend 2–4 Kohlenstoffatome, wie Diisopropyladipat, Diäthylsebacat oder Dibutylsebacat; flüssige Ester von aromatischen Dicarbonsäuren enthaltend 8 Kohlenstoffatome und von einwertigen Alkoholen, z.B. solchen enthaltend 2–4 Kohlenstoffatome, wie Diäthylphthalat oder Dibutylphthalat; flüssige Ester von Tricarbonsäuren, z.B. solchen enthaltend 6 Kohlenstoffatome, und einwertigen Alkoholen, z.B. solchen enthaltend 2–4 Kohlenstoffatome, wie die Ester von Citronensäure, z.B. Tributylcitrat oder Acetyltriäthylcitrat. Bevorzugte lipophile Lösungsmittel sind Diisopropyladipat, Isopropylmyristat und Isosorbiddimethyläther.

Geeignete flüssige hydrophile Lösungsmittel sind einwertige Alkohole z.B. solche enthaltend 2–3 Kohlenstoffatome, wie Äthanol, Isopropanol und n-Propanol; mehrwertige Alkohole, z.B. solche enthaltend 3–6 Kohlenstoffatome, wie Propandiol, Glycerin, Diäthylenglykol und Triäthylenglykol; Polyäthylenglykole, z.B. solche mit einem Molekulargewicht von 200–600; Polyhydroxyäthylen-polyhydroxypropylen-Kondensate, z.B. solche mit einem Molekulargewicht von 1900–3000, wie die verschiedenen im Handel befindlichen flüssigen Pluronic®-Typen der Wyandotte Chemicals Corp. Bevorzugt werden als flüssige hydrophile Lösungsmittel die genannten Polyäthylenglykol- und Polyhydroxyäthylen-polyhydroxypropylen-Kondensate.

Da die erfindungsgemässe pharmazeutische Zubereitung niedrigviskos sein soll, werden also nur solche flüssige Polyhydroxyäthylen-polyhydroxypropylen-Kondensate eingesetzt, die eine entsprechend niedere Viskosität besitzen, beispielsweise im Bereich von 250–700 mPas (cP).

Zweckmässigerweise besteht ein Teil des hydrophilen Lösungsmittels, vorzugsweise 50–70 Gew.-%, aus Äthanol.

Die erfindungsgemässen pharmazeutischen Zubereitungen sollen kein oder nur eine sehr geringe Menge Wasser enthalten. Ein Wassergehalt, wie er durch die Verwendung von normalen Äthanol gegeben ist, ist nicht störend. Im allgemeinen soll jedoch der Wassergehalt der erfindungsgemässen Zubereitungen nicht höher als 4 Gew.-%, vorzugsweise nicht höher als 2 Gew.-%, betragen.

Eine besonders bevorzugte erfindungsgemässe Zubereitung besteht aus etwa 10 Gew.-% Isosorbiddinitrat, etwa 27 Gew.-% Diisopropyladipat, etwa 27 Gew.-% Polyäthylenglykol 400 und etwa 36 Gew.-% Äthanol.

Die erfindungsgemässen pharmazeutischen Zubereitungen können als Sprühmittel verwendet werden. Vorzugsweise werden sie mittels einer mechanischen Dosierpumpe zerstäubt und auf die Haut gesprüht, möglicherweise nach dem Sprühen in die Haut eingerieben. Es resultiert eine sehr exakte Dosierung und die Lösung kann bei Einhaltung eines bestimmten Sprühabstandes auf eine genau definierte Hautfläche verteilt werden. Die besprühte Hautfläche bestimmt die Höhe des Blutspiegels des Wirkstoffes. Andere geeignete Anwendungsformen sind dem Fachmann geläufig.

Bisher konnte aus Lösungsmitteln, wie sie beispielsweise in der oben genannten US-PS 4 112 115 empfohlen werden, zwar eine gute Resorptionsquote des Wirkstoffes, jedoch keine Langzeitwirkung erreicht werden. Überraschend ist nun der erfindungsgemässe Befund, dass trotz des hohen Anteils an flüssigem lipophilen Lösungsmittel in den erfindungsgemässen Zubereitungen, welche alleine einen hohen und kurz andauernden Blutspiegel mit Isosorbiddinitrat erreichen lassen, bei Verschiebung des lipophilen Verhältnisses durch Zusatz von hydrophilen Lösungsmitteln eine Retardwirkung von über 12 Stunden erreicht wird. Dabei sind die Blutspiegel höher, als sie mit der Salbenzubereitung gemäss DE-OS 2 924 005 erzielt werden können.

Die erfindungsgemässen pharmazeutischen Zubereitungen können noch kleinere Mengen üblicher Zusatzstoffe, wie beispielsweise Konservierungsmittel, Antioxidationsmittel oder Parfümstoffe enthalten.

Die erfindungsgemässe Zubereitung wird so angewendet, dass eine tägliche Dosierung von etwa 0,5–6 mg, vorzugsweise 0,75–2 mg Isosorbiddinitrat je kg Körpergewicht resultiert. Die Dosierung kann jedoch auch höher oder niedriger liegen und wird vom Arzt im Einzelfall bestimmt.

In den folgenden Beispielen werden alle Zubereitungsschritte, sofern nicht anders angegeben, bei Raumtemperatur durchgeführt.

Beispiel 1:
1. 10 kg Isosorbiddinitrat
2. 27 kg Diisopropyladipat
3. 27 kg Polyäthylenglykol 400
4. 36 kg Äthanol

Die Rohstoffe 2 und 3 werden sorgfältig gemischt und in der Mischung Rohstoff 1 unter Umrühren gelöst.

Nach erfolgter Lösung wird Rohstoff 4 hinzugefügt und wieder durch Rühren homogen gemischt. Die Lösung wird filtriert.

Es werden je 50 g in Glasflaschen gefüllt, auf die ein Pumpzerstäuber mit einem Hub von 300 mg aufgesetzt wird.

Beispiel 2:
1. 10 kg Isosorbiddinitrat
2. 25 kg Diisopropyladipat
3. 29 kg Pluronic L 31® (ein Handelsprodukt der Wyandotte Chemicals Corp. bestehend aus Polyhydroxyäthylen-polyhydroxypropylen-Kondensat, Molekulargewicht 1050)
4. 36 kg Äthanol

Die Rohstoffe 2 und 3 werden sorgfältig durch Rühren gemischt und hierin Rohstoff 1 unter weiterem Rühren gelöst.

Nach erfolgter Lösung wird Rohstoff 4 hinzugefügt und mit der Lösung vermischt. Anschliessend wird filtriert und die Lösung in 50 ml Glasflaschen gefüllt, auf die ein Pumpzerstäuber aufgesetzt wird.

Beispiel 3:
1. 10 kg Isosorbiddinitrat
2. 26 kg Isosorbiddimethyläther
3. 64 kg Polyäthylenglykol 400

Rohstoff 1 wird unter Rühren in Rohstoff 2 gelöst. Nach vollständiger Lösung wird Rohstoff 3 unter weiterem Rühren hinzugefügt.

Die Lösung wird dann filtriert und in 50 ml Glasflaschen gefüllt, auf die Pumpzerstäuber aufgesetzt werden.

**Patentansprüche**

1. Pharmazeutische Zubereitung mit Retardwirkung für die topische Anwendung mit einem Gehalt an Isosorbiddinitrat als Wirkstoff und Lösungsmittel sowie üblichen Zusätzen, dadurch gekennzeichnet, dass sie
   a) ein flüssiges lipophiles Lösungsmittel für Isosorbiddinitrat und
   b) ein flüssiges hydrophiles Lösungsmittel für Isosorbiddinitrat
in einem Gewichtsverhältnis von a : b von 0,8–0,2, vorzugsweise von 0,5–0,3, enthält.

2. Pharmazeutische Zubereitung nach Anspruch 1, dadurch gekennzeichnet, dass sie Isosorbiddinitrat in einer Menge von 5–25 Gew.-%, vorzugsweise 8–15 Gew.-%, enthält.

3. Pharmazeutische Zubereitung nach Anspruch 1 oder 2, dadurch gekennzeichnet, dass das flüssige lipophile und das flüssige hydrophile Lösungsmittel solche sind, in denen Isosorbiddinitrat zu wenigstens 5 Gew.-%, vorzugsweise zu wenigstens 8 Gew.-%, löslich ist.

4. Pharmazeutische Zubereitung nach einem der vorhergehenden Ansprüche, dadurch gekennzeichnet, dass sie als flüssiges lipophiles Lösungsmittel Diisopropyladipat oder Isopropylmyristat oder Isosorbiddimethyläther enthält.

5. Pharmazeutische Zubereitung nach einem der vorhergehenden Ansprüche, dadurch gekennzeichnet, dass sie als flüssiges hydrophiles Lösungsmittel ein Polyäthylenglykol oder ein Polyhydroxyäthylen-Polyhydroxypropylen-Kondensat enthält.

6. Pharmazeutische Zubereitung nach einem der vorhergehenden Ansprüche, dadurch gekennzeichnet, dass ein Teil, vorzugsweise 50–70 Gew.-%, des hydrophilen Lösungsmittels aus Äthanol besteht.

7. Pharmazeutische Zubereitung nach einem der vorhergehenden Ansprüche, dadurch gekennzeichnet, dass ihr Wassergehalt nicht höher als 4 Gew.-%, vorzugsweise nicht höher als 2 Gew.-% ist.

8. Pharmazeutische Zubereitung nach einem der vorhergehenden Ansprüche, dadurch gekennzeichnet, dass sie aus etwa 10 Gew.-% Isosorbiddinitrat, etwa 27 Gew.-% Diisopropyladipat, etwa 27 Gew.-% Polyäthylenglykol 400 und etwa 36 Gew.-% Äthanol besteht.

9. Anwendung einer pharmazeutischen Zubereitung nach einem der vorhergenden Ansprüche als Sprühmittel.

10. Anwendung einer pharmazeutischen Zubereitung nach Anspruch 9 in einer mechanischen Dosierpumpe.

## Claims

1. Sustained action pharmaceutical formulation for topical use, containing isosorbide dinitrate as the active ingredient and solvents as well as customary additives, characterised in that it contains
a) a liquid lipophilic solvent for isosorbide dinitrate and
b) a liquid hydrophilic solvent for isosorbide dinitrate,
in a weight ratio of a : b of 0.8–0.2, preferably of 0.5–0.3.

2. Pharmaceutical formulation according to claim 1, characterised in that it contains isosorbide dinitrate in an amount of 5–25% by weight, preferably 8–15% by weight.

3. Pharmaceutical formulation according to claim 1 or 2, characterised in that the liquid lipophilic solvent and the liquid hydrophilic solvent are such that isosorbide dinitrate is soluble in them to an extent of at least 5% by weight, preferably of at least 8% by weight.

4. Pharmaceutical formulation according to one of the foregoing claims, characterised in that it contains, as the liquid lipophilic solvent, diisopropyl adipate or isopropyl myristate or isosorbide dimethyl ether.

5. Pharmaceutical formulation according to one of the foregoing claims, characterised in that it contains, as the liquid hydrophilic solvent, a polyethylene glycol or a polyhydroxyethylene/polyhydroxypropylene condensate.

6. Pharmaceutical formulation according to one of the foregoing claims, characterised in that a part, preferably 50–70% by weight, of the hydrophilic solvent consists of ethanol.

7. Pharmaceutical formulation according to one of the foregoing claims, characterised in that its water content is not greater than 4% by weight, preferably not greater than 2% by weight.

8. Pharmaceutical formulation according to one of the foregoing claims, characterised in that it consists of about 10% by weight of isosorbide dinitrate, about 27% by weight of diisopropyl adipate, about 27% by weight of polyethylene glycol 400 and about 36% by weight of ethanol.

9. Use of a pharmaceutical formulation according to one of the foregoing claims as a spraying agent.

10. Use of a pharmaceutical formulation according to claim 9 in a mechanical metering pump.

## Revendications

1. Préparation pharmaceutique à effet retardé pour l'application topique, renfermant une teneur en dinitrate d'isosorbide comme substance active et un solvant de même que des additifs classiques, caractérisée en ce qu'elle contient
a) un solvant lipophile liquide pour le dinitrate d'isosorbide et
b) un solvant hydrophile liquide pour le dinitrate d'isosorbide
dans un rapport en poids de a à b de 0,8–0,2, de préférence de 0,5–0,3.

2. Préparation pharmaceutique suivant la revendication 1, caractérisée en ce qu'elle contient du dinitrate d'isosorbide en une quantité de 5 à 25% en poids, de préférence de 8 à 15% en poids.

3. Préparation pharmaceutique suivant la revendication 1 ou 2, caractérisée en ce que le solvant liquide lipophile et le solvant liquide hydrophile sont des solvants dans lesquels de dinitrate d'isosorbide est soluble en proportion d'au moins 5% en poids et de préférence d'au moins 8% en poids.

4. Préparation pharmaceutique suivant l'une des revendications précédentes, caractérisée en ce qu'elle contient comme solvant lipophile liquide de l'adipate de diisopropyle ou du myristate d'isopropyle ou de l'éther diméthylique d'isosorbide.

5. Préparation pharmaceutique suivant l'une des revendications précédentes, caractérisée en ce qu'elle contient comme solvant hydrophile liquide un polyéthylèneglycol ou un produit de condensation polyhydroxyethylène-polyhydroxypropylène.

6. Préparation pharmaceutique suivant l'une des revendications précédentes, caractérisée en ce qu'une partie, de préférence de 50 à 70% en poids, du solvant hydrophile est constituée d'éthanol.

7. Préparation pharmaceutique suivant l'une des revendications précédentes, caractérisée en ce que sa teneur en eau ne dépasse pas 4% en poids, et de préférence ne dépasse pas 2% en poids.

8. Préparation pharmaceutique suivant l'une des revendications précédentes, caractérisée en ce qu'elle est constituée d'environ 10% en poids de dinitrate d'isosorbide, d'environ 27% en poids d'adipate de diisopropyle, d'environ 27% en poids de polyéthylèneglycol 400 et d'environ 36% en poids d'éthanol.

9. Utilisation d'une préparation pharmaceutique selon l'une des revendications précédentes comme composition pulvérisable.

10. Utilisation d'une préparation pharmaceutique suivant la revendication 9 dans une pompe mécanique à dose.